# EUROPÄISCHE PATENTSCHRIFT

(11) **EP 1 574 560 B1**
(45) Veröffentlichungstag und Bekanntmachung des Hinweises auf die Patenterteilung: **12.01.2011**
(21) Anmeldenummer: 05003714.2
(22) Anmeldetag: 22.02.2005
(51) Int. Cl.: C07C 59/60, C07C 51/367, C07C 59/125

(54) **Verfahren zur lösungsmittelfreien Herstellung von Ethercarbonsäuren mit niedrigem Restsalzgehalt**
Process for solventfree production of ether carboxylic acids with a low rest salt content
Procédé sans solvant de préparation d' acides ether carboxyliques à faible teneur en sel

(30) Priorität: 04.03.2004 DE 102004010505
(43) Veröffentlichungstag der Anmeldung: 14.09.2005
(73) Patentinhaber: Clariant Produkte (Deutschland) GmbH, 65929 Frankfurt am Main (DE)
(72) Erfinder: Leinweber, Dirk, Dr., 65824 Schwalbach (DE); Dahlmann, Uwe, Dr., 69126 Heidelberg (DE); Kupfer, Rainer, Dr., 65795 Hattersheim (DE)

(56) Entgegenhaltungen:
- EP-A- 1 061 064

## Beschreibung

Die vorliegende Erfindung betrifft ein Verfahren zur lösungsmittelfreien Herstellung von Ethercarbonsäuren mit niedrigem Restsalzgehalt.

Ethercarbonsäuren, d.h. organische Carbonsäuren, die neben der Carboxylfunktion eine oder mehrere Etherbrücken tragen, bzw. deren Alkali- oder Aminsalze, sind als milde Detergenzien mit hohem Kalkseifendispergiervermögen bekannt. Sie finden sowohl in Waschmittel- und Kosmetikformulierungen, aber auch in technischen Anwendungen, w.z.B. Metallbearbeitungsflüssigkeiten und Kühlschmiermittel, Verwendung.

Ethercarbonsäuren (ECS) werden gemäß dem Stand der Technik entweder durch Alkylierung von Alkohol- oder Fettalkoholoxethylaten oder -oxpropylaten mit Chloressigsäurederivaten (Williamsonsche Ethersynthese/Carboxyalkylierung) oder aus den gleichen Ausgangsprodukten durch Oxidation mit verschiedenen Reagenzien (Luftsauerstoff, Hypochlorit, Chlorit) unter Katalyse mit verschiedenen Katalysatoren hergestellt. Die Williamsonsche Ethersynthese stellt vor allem aufgrund der Kosten- Wirkung- Beziehung das technisch geläufigste Verfahren für die Herstellung von ECS dar, jedoch besitzen durch dieses Verfahren hergestellte Produkte noch gravierende Mängel in Bezug auf die Handhabbarkeit für den Anwender, wie beispielsweise Löslichkeitsverhalten, Aggregatzustand bei niedrigen Temperaturen und insbesondere der recht hohe Restsalzgehalt, der zu verstärkter Korrosion führen kann.

Diese Mängel sind im wesentlich auf verfahrensbedingte Nebenbestandteile zurückzuführen. So werden trotz Verwendung von Überschüssen des entsprechenden Chloressigsäurederivats nur Umsätze von ca. 70-85 % erreicht, so dass Restmengen an Oxethylat und Fettalkohol, der dem Oxethylat zugrunde liegt, im Endprodukt verbleiben. Des weiteren entstehen durch den zu verwendenden Überschuss des Chloressigsäurederivats Folgeprodukte, wie beispielsweise Glycolsäure, Diglycolsäure und deren Derivate, die eine wesentliche Ursache für die Alterung der Produkte sind und gegebenenfalls Probleme beim Löslichkeitsverhalten hervorrufen können.

Ein weiterer gravierender Nachteil der Williamson- Synthese besteht in der hohen Belastung der Reaktionsprodukte durch Natriumchlorid (Gehalt ca. 1 %), das in wässrigen Lösungen eine wesentliche Ursache für Lochfraß-Korrosion darstellt. Der Natriumchlorid-Gehalt lässt sich zwar durch Waschprozesse mit gesättigten wässrigen Lösungen andere Metallsalze reduzieren. Allerdings wird hierdurch nur der Natriumchlorid-Gehalt durch den Gehalt des entsprechenden Metallsalzes der Waschphase substituiert. Somit kann also kein Reaktionsprodukt hergestellt werden, das verminderte korrosive Eigenschaften aufweist.

DE-A-199 28 128 offenbart ein Verfahren zur Herstellung von Ethercarbonsäuren mit niedrigem Restalkoholgehalt, indem Fettalkohole zunächst unter Einsatz nichtkatalytischer Mengen an Alkalikatalysator (NaOH, KOH, Alkoholate über 5 Mol-%) mit Alkylenoxiden umgesetzt werden, und die resultierenden, stark alkalischen Reaktionsmischungen, die aus einem Gemisch von oxethylierten Alkoholen und Alkoholaten verschiedener Polyalkylenglykolether bestehen, anschließend in einer klassischen Williamson-Synthese mit Natriumchloracetat in die entsprechende Ethercarbonsäure überführt werden. Durch dieses Verfahren wird zwar der Restgehalt an Fettalkohol in der Ethercarbonsäure ohne spezielle Katalysatoren verringert, jedoch wird der hohe Restsalzgehalt der Produktphase nicht reduziert.

In EP-A-0 897 906 wird ein Verfahren zur Herstellung von Polyoxyalkylencarbonsäuren beschrieben. Hierbei werden die beschriebenen Ethercarbonsäuren nach dem technisch üblichen Verfahren hergestellt. Allerdings wird einerseits der Carboxyalkylierungsschritt unter Zuhilfenahme eines Lösungsmittels und andererseits zur Aufreinigung der organischen Produktphase ein Waschprozess mit einer wässrigen Lösung eines Metallsalzes durchgeführt. Hierdurch soll eine hochreine Ethercarbonsäure erhalten werden. Sowohl die Verwendung eines Lösungsmittels als auch der beschriebene Waschprozess ist ökonomisch und ökologisch nachteilig. Zudem führt der beschriebene Waschprozess lediglich zu einer Substitution des aus dem Carboxyalkylierungsschritt stammenden Natriumchlorids durch ein anderes Salz. Daher kann das organische Rohprodukt mit einem derartigen Waschprozess nicht von Metallsalzen befreit werden. Die beschriebenen Ethercarbonsäuren sollen hochrein sein, allerdings werden keine Restsalzgehalte offenbart, noch werden Entscheidungskriterien genannt, um einen niedrigen Restsalzgehalt festzustellen.

Es bestand daher die Aufgabe, ein ökonomisch und ökologisch vorteilhaftes Verfahren zur Herstellung von Ethercarbonsäuren zu entwickeln, die sich durch einen besonders niedrigen Restsalzgehalt auszeichnen.

Überraschenderweise wurde gefunden, dass die nach einer lösungsmittelfreien Carboxyalkylierung erhaltenen Ethercarbonsäuren durch direktes Abdestillieren des in der organischen Produktphase enthaltenden Restwassers unter vermindertem Druck und anschließender Filtration des ausfallenden Metallsalzes (i.d.R. NaCl) einen niedrigen Restsalzgehalt von < 0,20 % aufweisen, der direkt durch Leitfähigkeitsmessungen feststellbar ist.

Gegenstand der Erfindung ist daher ein Verfahren zur Herstellung von Verbindungen der Formel (1) 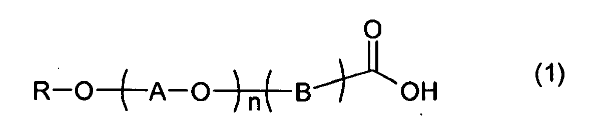 wobei
- A: C₂- bis C₄-Alkylen,
- B: C₁- bis C₄-Alkylen
- n: eine Zahl von 1 bis 100, und
- R: C₁- bis C₃₀-Alkyl, C₂- bis C₃₀-Alkenyl, oder C₆- bis C₃₀-Aryl
bedeuten, indem man
a) ein basisches Gemisch von oxalkylierten Alkoholen der Formel 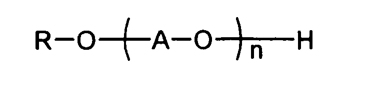 und deren Alkoholaten mit einer C₂- bis C₅-Chlorcarbonsäure in Abwesenheit eines Lösungsmittels alkyliert
b) das so erhaltene Ethercarbonsäuresalz durch Zugabe einer Säure, wobei der pH-Wert auf 3 oder weniger eingestellt wird, in die freie Ethercarbonsäure überführt,
c) die so erhaltene Ethercarbonsäure ohne Waschen durch Destillation unter vermindertem Druck bis zu einem Restwassergehalt von <0,30 % vom enthaltenen Wasser befreit, und
d) die ausfallenden Metallsalze durch Filtration entfernt, so dass das erhaltene Produkt einen Restsalzgehalt von <0,2 % aufweist.

A bedeutet vorzugsweise Propylen oder Ethylen, insbesondere Ethylen. In einer weiteren bevorzugten Ausführungsform der Erfindung steht die Gruppe -(A-O)ₙ- für eine gemischte Alkoxygruppe, die Ethylen-, Propylen- und Butylenreste enthalten kann. Handelt es sich um eine gemischte Alkoxygruppe, so liegt das Verhältnis der vom Ethylenoxid abgeleiteten Gruppen zu den von Propylen- oder Butylenoxid abgeleiteten Gruppen vorzugsweise zwischen 10:1 und 1:1.

n steht vorzugsweise für eine Zahl zwischen 2 und 70, insbesondere 3 bis 50.

B steht vorzugsweise für eine geradkettige Alkylengruppe, insbesondere für Methylen. B kann auch für eine verzweigte Alkylengruppe mit 3 oder 4 Kohlenstoffatomen stehen.

R bedeutet in einer bevorzugten Ausführungsform einen C₈-C₂₄, insbesondere einen C₁₂-C₁₈-Alkyl- oder Alkenylrest. Steht R für einen aromatischen Rest, so ist ein Phenylrest mit Alkylsubstitution zwischen 4 und 12 Kohlenstoffatomen bevorzugt.

X kann in einer bevorzugten Ausführungsform für Wasserstoffionen stehen. In einer weiteren bevorzugten Ausführungsform steht X für Alkali- oder Erdalkaliionen, insbesondere Lithium, Natrium, Kalium, Magnesium, oder Calcium.

In einer weiteren bevorzugten Ausführungsform werden als Kationen Ammoniumionen der Formel NR¹R²R³R⁴ verwendet, wobei R¹, R², R³ und R⁴ unabhängig voneinander H, C₁- bis C₂₂-Alkyl, C₆- bis C₁₈-Aryl, C₇- bis C₂₂-Alkylaryl und/oder C₁- bis C₂₂-Alkenyl bedeuten können. Die Reste R¹, R², R³ und R⁴ können Heteroatome wie N, P, O, S enthalten. Die Ammoniumreste können Monoalkylammonium-, Dialkylammonium-, Trialkylammonium- oder Tetraalkylammoniumreste sein, wobei die Alkylsubstituenten unabhängig voneinander mit bis zu 3 Hydroxygruppen besetzt sein können. Vorzugsweise steht X für Ammoniumreste, die einen, zwei, drei oder vier C₂- bis C₁₀-Alkylreste tragen. In einer weiteren bevorzugten Ausführungsform können einer, zwei oder drei der Reste R¹ bis R⁴ alkoxyliert sein.

Geeignete Amine für die Herstellung von Ammoniumkationen X sind Monoamine mit primärer oder sekundärer Aminofunktion wie Methylamin, Ethylamin, Butylamin, Laurylamin, Cocosfettamin, Stearylamin, Dimethylamin, Diethylamin, Dibutylamin, aber auch Di- und Polyamine wie z.B. 3-Dimethylaminopropylamin, 3-Diethylaminopropylamin, 3-Morpholinopropylamin, Diethylentriamin, Triethylentetramin oder Tetraethylenpentamin.

Geeignete Aminoalkohole für die Herstellung von Ammoniumkationen X sind beispielsweise N,N-Dimethylaminoethanol, N,N-Diethylaminoethanol, N,N-Dibutylaminoethanol, 3-Dimethylaminopropanol, N-Hydroxyethylmorpholin, Monoethanolamin, Diethanolamin, Triethanolamin, 3-Aminopropanol, Isopropanolamin, 2-(2-Aminoethoxy)ethanol und Cyclohexylamino-N,N-diethanol.

Als Basisfettalkohol für das hier beschriebene Verfahren eignen sich lineare oder verzweigte, gesättigte oder ungesättigte Fettalkohole mit 1-30 C-Atomen sowie Alkylphenole mit einem C₁-C₂₀ Alkylrest, bevorzugt sind C₆-C₂₂ Fettalkohole.

Diese können gemäß des Standes der Technik mit Alkylenoxiden, z.B. Ethylenoxid, Propylenoxid, Butylenoxid oder Mischungen verschiedener solcher Alkylenoxide umgesetzt werden, wobei Ethylenoxid oder Mischungen aus Ethylenoxid und Propylenoxid bevorzugt sind. Bezogen auf Fettalkohol werden 1-100 mol Alkylenoxid beaufschlagt, bevorzugt 3-50 mol. Die Reaktionstemperaturen liegen hierbei bei ca. 80-160°C.

Im anschließenden Reaktionsschritt wird die Alkoholat/Alkoholoxethylat-Mischung mit einem Chlorcarbonsäurederivat und einer Base, bevorzugt trockenem Chloressigsäure-Natriumsalz und Natriumhydroxid umgesetzt. Dies kann geschehen, indem man die Oxethylat/Alkoholat-Mischung mit 100-150 Mol-% Natriumchloracetat bei 30-100°C umsetzt und gleichzeitig oder nacheinander mit festem Natriumhydroxid oder Kaliumhydroxid versetzt, so dass die Summe aus der in der Oxethylat-/Alkoholatmischung vorliegenden Base und der zusätzlich zugegebenen Basenmenge der Menge an Natriumchloracetat entspricht.

Nach der Carboxyalkylierung kann das Ethercarbonsäure-Alkalisalz durch Absäuerung auf pH < 3 mit einer beliebigen Säure in die freie Ethercarbonsäure überführt werden. Die wässrige Phase wird abgetrennt und die organische Phase direkt, d.h. ohne Verwendung eines Waschprozesses, durch Destillation unter vermindertem Druck vom enthaltenen Restwasser (ca. 5-15 %) befreit. Hierdurch wird das enthaltende Restsalz ausgefällt, welches anschließend in einem Filtrationsschritt entfernt wird. Die so erhaltene salzarme Ethercarbonsäure kann anschließend wieder mit einer entsprechenden Menge deionisierten Wassers versetzt werden, um vergleichbare physikalische Eigenschaften analog der salzhaltigen, wässrigen Roh-Ethercarbonsäure zu erzielen. Im Vergleich zu den Roh-Ethercarbonsäuren, die einen Salzgehalt von 0,60-1,50 % aufweisen, enthalten die durch den beschriebenen Prozess hergestellten Ethercarbonsäuren nur noch Metallsalze von <0,20 %.

Wie die folgenden Beispiele zeigen, können nach dem hier offenbarten Verfahren Ethercarbonsäuren mit sehr niedrigem Restsalzgehalt von < 0,20 % hergestellt werden. Solche Ethercarbonsäuren weisen im allgemeinen eine Leitfähigkeit von < 15 µS/cm auf.

### Beispiele

### Beispiel 1

### Oleylalkohol + 10 EO - ECS (Standardverfahren = Vergleichsbeispiel 1)

In einer 2 I Rührapparatur wurden 412 g (0,57 mol) Oleylalkohol + 10 EO (z.B. Genapol O 100) unter Stickstoffspülung vorgelegt und auf 40°C erwärmt. Dann wurden unter gutem Rühren 92,0 g (0,79 mol) Chloressigsäure-Natriumsalz eingetragen und die Reaktionsmischung auf 50°C erwärmt. Anschließend wurden insgesamt 35,0 g (0,88 mol) Natriumhydroxid-Microprills portionsweise so zugegeben, dass die Innentemperatur 55°C nicht übersteigt. Nach jeder Zugabe wurde jeweils 30 min, nach der letzten Zugabe 2 h bei 70°C nachgerührt. Die Reaktionsmischung wurde dann auf 90°C erwärmt und dann warme Salzsäure (8-32 %ig) zulaufen lassen, bis ein pH-Wert < 3 erreicht ist. Die Reaktionsmischung wurde dann gleichmäßig durchmischt, auf ca. 100°C erhitzt und in ein beheizbares Trenngefäß mit Rührung und Bodenventil überführt. Phasentrennung erfolgte ohne Rühren bei einer Temperatur von ca. 100-110°C. Nach Abtrennung der wässrigen Unterphase wurden 448 g Produkt als hell-gelbe Flüssigkeit erhalten.

### Beispiel 2

### Oleylalkohol + 10 EO - ECS (Entfernen des Restwassers und Filtration)

Herstellung der Oleylalkohol + 10 EO - ECS erfolgte nach Beispiel 1. Nach Abtrennung der wässrigen Unterphase wurde das enthaltende Restwasser (KF = 8,9 %) der organischen Produktphase bei einem verminderten Druck von 200-50 mbar und einer Temperatur von 100°C destillativ entfernt. Nach Abkühlen der Produktphase auf 50-60°C wurde ausgefallenes Natriumchlorid mittels Filtration entfernt. Es wurden 395 g salzarmes Produkt als hellgelbe viskose Flüssigkeit erhalten. Zum Angleich der physikalischen Eigenschaften wurden dem salzarmen Produkt anschließend wieder 8,9 % deionisiertes Wasser zugemischt.

### Beispiel 3

### n-Octanol + 8 EO (Standardverfahren = Vergleichsbeispiel 2)

In einer 2 I Rührapparatur wurden 362 g (0,75 mol) n-Octanol + 8 EO (z.B. Genapol O 080) unter Stickstoffspülung vorgelegt und auf 40°C erwärmt. Dann wurden unter gutem Rühren 104,8 g (0,90 mol) Chloressigsäure-Natriumsalz eingetragen und die Reaktionsmischung auf 50°C erwärmt. Anschließend wurden insgesamt 39,8 g (1,00 mol) Natriumhydroxid-Microprills portionsweise so zugegeben, dass die Innentemperatur 55°C nicht übersteigt. Nach jeder Zugabe wurde jeweils 30 min, nach der letzten Zugabe 2 h bei 70°C nachgerührt. Die Reaktionsmischung wurde dann auf 90°C erwärmt und dann warme Salzsäure (35 %ig) zulaufen lassen bis ein pH-Wert < 3 erreicht ist. Die Reaktionsmischung wurde dann gleichmäßig durchmischt, auf ca. 100°C erhitzt und in ein beheizbares Trenngefäß mit Rührung und Bodenventil überführt. Die Phasentrennung erfolgte ohne Rührung bei einer Temperatur von ca. 100-110°C. Nach Abtrennung der wässrigen Unterphase wurden 405 g Produkt als hell-gelbe Flüssigkeit erhalten.

### Beispiel 4

### n-Octanol + 8 EO (Entfernen des Restwassers und Filtration)

Herstellung der n-Octanol + 8 EO - ECS erfolgte nach Beispiel 3. Nach Abtrennung der wässrigen Unterphase wurde das enthaltende Restwasser (KF = 10,2 %) der organischen Produktphase bei einem verminderten Druck von 200-50 mbar und einer Temperatur von 100°C destillativ entfernt. Nach Abkühlen der Produktphase auf 50-60°C wurde ausgefallenes Natriumchlorid mittels Filtration entfernt. Es wurden 355 g salzarmes Produkt als hell-gelbe viskose Flüssigkeit erhalten. Zum Angleich der physikalischen Eigenschaften wurden dem salzarmen Produkt anschließend wieder 10,2 % deionisiertes Wasser zugemischt.

### Beispiel 5

### Oleylalkohol + 10 EO - ECS

### (Standardprozess + Waschen mit Natriumchlorid-Lösung = Vergleichsbeispiel 3)

In einer 2 I Rührapparatur wurden 412 g (0,57 mol) Oleylalkohol + 10 EO (z.B: Genapol O 100) unter Stickstoffspülung vorgelegt und auf 40°C erwärmt. Dann wurden unter gutem Rühren 92,0 g (0,79 mol) Chloressigsäure-Natriumsalz eingetragen und die Reaktionsmischung auf 50°C erwärmt. Anschließend wurden insgesamt 35,0 g (0,88 mol) Natriumhydroxid-Microprills portionsweise so zugegeben, dass die Innentemperatur 55°C nicht übersteigt. Nach jeder Zugabe wurde jeweils 30 min, nach der letzten Zugabe 2 h bei 70°C nachgerührt. Die Reaktionsmischung wurde dann auf 90°C erwärmt und dann warme Salzsäure (8-32%ig) zulaufen lassen, bis ein pH-Wert < 3 erreicht ist. Die Reaktionsmischung wurde dann gleichmäßig durchmischt, auf ca. 100°C erhitzt und in ein beheizbares Trenngefäß mit Rührung und Bodenventil überführt. Phasentrennung erfolgte ohne Rühren bei einer Temperatur von ca. 100-110°C. Nach Abtrennung der wässrigen Unterphase wurde das Rohprodukt zweimal mit einer gesättigten wässrigen Natriumchlorid-Lösung gewaschen. Nach Abtrennung der Waschphase wurden 439 g Produkt als hell-gelbe Flüssigkeit erhalten.

**Tabelle 1: Kennzahlen der Ethercarbonsäuren**

| Beispiel | Gehalt NaCl [%] | Leitfähigkeit [µS/cm] | Leitfähigkeit [µS/cm] (0,05 M in Wasser) | SZ [mg KOH/g] | VZ [mg KOH/g] |
|---|---|---|---|---|---|
| 1 (V) | 0,87 | 47,0 | 1182 | 72,2 | 73,6 |
| 2 | 0,09 | 4,93 | 726 | 71,9 | 73,8 |
| 3 (V) | 0,95 | 93,2 | 2480 | 105,2 | 107,5 |
| 4 | 0,12 | 11,4 | 1362 | 104,9 | 107,4 |
| 5 (V) | 0,78 | 46,8 | 1175 | 72,1 | 73,6 |

| | | | | | |
|---|---|---|---|---|---|
| (SZ = Säurezahl, VZ = Verseifungszahl) | | | | | |

Wie aus der Tabelle 1 ersichtlich ist, zeichnen sich die durch das hier offenbarte Verfahren hergestellten Ethercarbonsäuren durch einen besonders niedrigen Salzgehalt (Natriumchlorid) aus, der sich durch eine deutlich geringere Leitfähigkeit im Vergleich zu den Ethercarbonsäuren aus dem Standard-Prozess manifestiert. Weiterhin ergibt sich aus dem Vergleich der gefundenen Säure- und Verseifungszahlen, dass durch den beschriebenen Prozess kaum vermehrte (unerwünschte) Esterbildung erfolgt.

Durch einen Waschprozess nach dem Stand der Technik kann der Natriumchlorid-Gehalt von Ethercarbonsäuren zwar geringfügig gesenkt werden (siehe Vergleichsbeispiel 3), allerdings sind Salzgehalte von unter 0,2 % so nicht erreichbar.

Verwendung der erfindungsgemäßen Verbindungen als Korrosionsinhibitor für wassermischbare Kühlschmiermittel, Reinigungsflüssigkeiten, und für Oberflächenbehandlungen.

Die Korrosionsschutzprüfung erfolgte in Anlehnung an die DIN-Norm 51360, Teil 2 (Filterpapiertest) und dient der Beurteilung der Korrosion von Eisenmetall. Als Maß für die Korrosion dienen Art und Anzahl der Korrosionsabzeichnungen auf einem Rundfilter, die durch Einwirken eines mit Wasser gemischten Kühlschmierstoffes (KSS) auf genormte Graugussspäne (Spangröße: 3 bis 6 mm²) entstehen. Die Beurteilung erfolgt durch Sichtprüfung und Einstufung des Korrosionsgrades (1 bis 4) nach Vergleichstabelle.

Die zu prüfenden Produkte wurden für die Untersuchungen zum Korrosionsschutz mit Triethanolamin (TEA) unter Bildung des entsprechenden Ammoniumsalzes auf pH 9,0 eingestellt.

**Tabelle 2: Korrosionsschutzprüfung nach DIN (Filterpapiertest), Angabe in Korrosionsgraden 1 bis 4 nach Vergleichstabelle DIN-Norm 51360, Teil 2 (Filterpapiertest), Konzentrationen in Gew.-%**

| ECS aus Beispiel | NaCl-Gehalt [%] | Konzentration der Ethercarbonsäure (ECS) | | |
|---|---|---|---|---|
| | | 3% | 4% | 5% |
| 1(V) | 0,87 | 4 | 3-4 | 3 |
| 2 | 0,09 | 2 | 1-2 | 1 |
| 3(V) | 0,95 | 3-4 | 3-4 | 3 |
| 4 | 0,12 | 2-3 | 1-2 | 1 |
| 5(V) | 0,78 | 4 | 3-4 | 3 |

Wie aus der Tabelle 2 ersichtlich ist, führt der niedrige Salzgehalt der Ethercarbonsäuren zu einem deutlich verbesserte Korrosionsschutzverhalten der erfindungsgemäßen Ethercarbonsäuren.

## Patentansprüche

1. Verfahren zur Herstellung von Verbindungen der Formel (1) 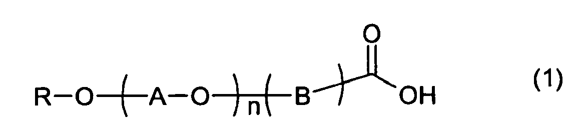 wobei
A C₂- bis C₄-Alkylen,
B C₁- bis C₄-Alkylen
n eine Zahl von 1 bis 100, und
R C₁- bis C₃₀-Alkyl, C₂- bis C₃₀-Alkenyl, oder C₆- bis C₃₀-Aryl bedeuten, indem man
a) ein basisches Gemisch von oxalkylierten Alkoholen der Formel 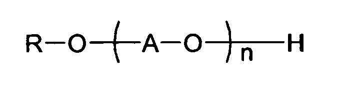 und deren Alkoholaten mit einer C₂- bis C₅-Chlorcarbonsäure in Abwesenheit eines Lösungsmittels alkyliert
b) das so erhaltene Ethercarbonsäuresalz durch Zugabe einer Säure, wobei der pH-Wert auf 3 oder weniger eingestellt wird, in die freie Ethercarbonsäure überführt,
c) die so erhaltene Ethercarbonsäure ohne Waschen durch Destillation unter vermindertem Druck bis zu einem Restwassergehalt von < 0,30 % vom enthaltenen Wasser befreit, und
d) die ausfallenden Metallsalze durch Filtration entfernt, so dass das erhaltene Produkt einen Restsalzgehalt von < 0,2 % aufweist.

2. Verfahren gemäß Anspruch 1, wobei A für Propylen oder Ethylen steht.

3. Verfahren gemäß Anspruch 1 und/oder 2, wobei n für eine Zahl zwischen 2 und 70 steht.

4. Verfahren gemäß einem oder mehreren der Ansprüche 1 bis 3, wobei B für eine Methylengruppe steht.

5. Verfahren gemäß einem oder mehreren der Ansprüche 1 bis 4, wobei R für einen C₈- bis C₂₄-Alkyl- oder Alkenylrest steht.

6. Verfahren gemäß einem oder mehreren der Ansprüche 1 bis 5, wobei in Schritt b) Salzsäure oder Schwefelsäure zur Absäuerung verwendet wird.

## Claims

1. A process for preparing compounds of the formula (1) 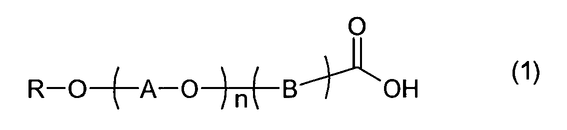 where
A is C₂- to C₄-alkylene,
B is C₁- to C₄-alkylene,
n is a number from 1 to 100, and
R is C₁- to C₃₀-alkyl, C₂- to C₃₀-alkenyl, or C₆- to C₃₀-aryl,
by
a) alkylating a basic mixture of oxoalkylated alcohols of the formula 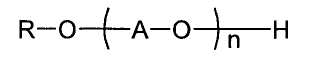 and alkoxides thereof with a C₂- to C₅-chlorocarboxylic acid in the absence of a solvent,
b) converting the thus obtained ethercarboxylic acid salt to the free ethercarboxylic acid by adding an acid, the pH being adjusted to 3 or less,
c) freeing the thus obtained ethercarboxylic acid, without washing, of water present down to a residual water content of < 0.30% by distillation under reduced pressure, and
d) removing the precipitated metal salts by filtration, so that the resulting product has a residual salt content of < 0.2%.

2. The process as claimed in claim 1, wherein A is propylene or ethylene.

3. The process as claimed in claim 1 and/or 2, wherein n is a number between 2 and 70.

4. The process as claimed in one or more of claims 1 to 3, wherein B is a methylene group.

5. The process as claimed in one or more of claims 1 to 4, wherein R is a C₈- to C₂₄-alkyl or -alkenyl radical.

6. The process as claimed in one or more of claims 1 to 5, wherein hydrochloric acid or sulfuric acid is used for acidification in step b).

## Revendications

1. Procédé pour la préparation de composés de formule (1), 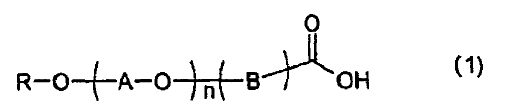 où
A signifie C₂-C₄-alkylène,
B signifie C₁-C₄-alkylène,
n vaut un nombre de 1 à 100 et
R signifie C₁-C₃₀-alkyle, C₂-C₃₀-alcényle, ou C₆-C₃- aryle en ce qu'on
a) alkyle un mélange basique d'alcools oxalkylés de formule 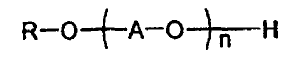 et leurs alcoolates avec un acide C₂-C₅-chlorocarboxylique en l'absence d'un solvant
b) transforme le sel d'acide éthercarboxylique ainsi obtenu par addition d'un acide, le pH étant réglé à 3 ou moins, en acide éthercarboxylique libre,
c) libère l'acide éthercarboxylique ainsi obtenu de l'eau contenue sans lavage, par distillation sous pression réduite jusqu'à une teneur résiduelle en eau < 0,30%, et
d) élimine les sels métalliques qui se séparent par précipitation par filtration, de manière telle que le produit obtenu présente une teneur résiduelle en sel < 0,2%.

2. Procédé selon la revendication 1, où A représente propylène ou éthylène.

3. Procédé selon la revendication 1 et/ou 2, où n représente un nombre entre 2 et 70.

4. Procédé selon l'une ou plusieurs des revendications 1 à 3, où B représente un groupe méthylène.

5. Procédé selon l'une ou plusieurs des revendications 1 à 4, où R représente un radical C₈-C₂₄-alkyle ou un radical C₈-C₂₄-alcényle.

6. Procédé selon l'une ou plusieurs des revendications 1 à 5, où, dans l'étape b), on utilise de l'acide chlorhydrique ou sulfurique pour l'acidification.
